# EUROPÄISCHE PATENTSCHRIFT

(11) **EP 2 275 037 B1**
(45) Veröffentlichungstag und Bekanntmachung des Hinweises auf die Patenterteilung: **06.06.2012**
(21) Anmeldenummer: 10168815.8
(22) Anmeldetag: 08.07.2010
(51) Int. Cl.: A61B 17/068, A61B 17/00

(54) **Druckgasbetriebenes Instrument**
Instrument operated using pressurised gas
Instrument entraîné par gaz comprimé

(30) Priorität: 17.07.2009 DE 102009033524
(43) Veröffentlichungstag der Anmeldung: 19.01.2011
(73) Patentinhaber: AESCULAP AG, 78532 Tuttlingen (DE)
(72) Erfinder: Scholten, Thomas, 78532, Tuttlingen (DE); Mayenberger, Rupert, 78239, Rielasingen (DE)
(74) Vertreter: Hoeger, Stellrecht & Partner Patentanwälte

(56) Entgegenhaltungen:
- DE-B3-102006 024 759
- DE-U1-202006 008 404
- DE-U1-202007 006 801
- US-A- 3 537 481
- US-A- 4 357 940

## Beschreibung

Die Erfindung betrifft ein druckgasbetriebenes Instrument, insbesondere ein medizinisches Instrument, mit einem Anschluss für eine Druckgaspatrone, an dem die Druckgaspatrone abgedichtet mit einem Zufuhrkanal für aus der Druckgaspatrone ausströmendes Druckgas verbindbar ist, mit einem Anschlussschacht, in den die Druckgaspatrone mit ihrem Anschlussbereich einsetzbar ist, und mit einem gegenüber der Innenwand des Anschlussschachtes abgedichteten Dichtstück, das in dem Anschlussschacht zwischen einer eingeschobenen Dichtstellung, in der es abdichtend an die Stirnfläche der in den Anschlussschacht eintauchenden Druckgaspatrone angedrückt wird, und einer ausgeschobenen Ruhestellung verschiebbar gelagert ist.

In der DE 10 2006 024 759 B3 ist ein druckgasbetriebenes Instrument beschrieben, bei dem eine Druckgaspatrone in eine Kammer eines Handgriffes einlegbar ist und dadurch mit einer Druckgasleitung 8 abgedichtet verbunden ist. In einer zylindrischen Ventilkammer, die sich an die Druckgasleitung 8 anschließt, befindet sich ein in der Ventilkammer verschiebbar gelagerter Schließkörper 22, der sich die Druckgasleitung 8 verschließend an eine Eintrittsöffnung der Druckgasleitung 8 in die zylindrische Ventilkammer anlegen kann. Die Stellung der Druckgaspatrone, ..kammer? des Handgriffes hat dabei keinerlei Einfluss auf die Stellung des Schließkörpers 22 in der zylindrischen Ventilkammer.

Ein druckgasbetriebenes Instrument, bei dem eine Druckgaspatrone in der eingangs beschriebenen Weise an das Instrument anschließbar ist, ist aus der DE 20 2007 006 801 U1 bekannt. Dabei wird durch ein verschiebliches Dichtstück eine dichte Verbindung zu dem Anschlussbereich der Druckgaspatrone hergestellt, und dieses Dichtstück hat eine selbstverstärkende Wirkung, da aufgrund unterschiedlicher Querschnittsflächen der Druck des aus der Druckgaspatrone ausströmenden Druckgases eine Differenzkraft auf das Dichtstück ausübt, die das Dichtstück gegen den Anschlussbereich der Druckgaspatrone drückt und dadurch die Dichtwirkung in diesem Bereich verstärkt.

Wenn man nach dem Betrieb des druckgasbetriebenen Instrumentes die Druckgaspatrone entfernt, ist diese in der Regel noch teilweise gefüllt. Beim Abziehen von dem Dichtstück leert sich daher die Druckgaspatrone sehr plötzlich unter lautem Zischen, und dies ist für den Benutzer sehr unangenehm.

Es ist Aufgabe der Erfindung, diese unangenehme plötzliche Entleerung der Druckgaspatrone bei deren Entfernung aus dem Instrument zu verhindern.

Diese Aufgabe wird bei einem druckgasbetriebenen Instrument der eingangs beschriebenen Art erfindungsgemäß dadurch gelöst, dass der Anschlussschacht eine Belüftungsöffnung aufweist, die mit dem stromabwärts des Dichtstückes gelegenen Abschnitt des Anschlussschachtes in Verbindung steht, wenn das Dichtstück bis zu einer zwischen Dichtstellung und Ruhestellung liegenden Belüftungsstellung aus seiner Dichtstellung entfernt ist, während das Dichtstück diesen Abschnitt in der Dichtstellung von der Belüftungsöffnung abtrennt.

So lange die Druckgaspatrone in den Anschlussschacht eingesetzt ist und das Dichtstück dadurch in die Dichtstellung verschiebt, bleibt der stromabwärts des Dichtstückes gelegene Abschnitt des Anschlussschachtes von der Belüftungsöffnung getrennt. Sobald jedoch das Dichtstück beim Herausziehen der Druckgaspatrone aus dem Anschlussschacht in Richtung auf die Ruhestellung verschoben wird, gelangt sie bei dieser Verschiebung in eine Belüftungsstellung, bei der das Dichtstück eine Verbindung zwischen der Belüftungsöffnung einerseits und dem stromabwärts des Dichtstückes gelegenen Abschnitt des Anschlussschachtes andererseits herstellt, das heißt über diese Belüftungsöffnung erfolgt eine Entleerung dieses stromabwärts des Dichtstückes gelegenen Abschnitts und damit auch der noch abgedichtet am Dichtstück anliegenden Druckgaspatrone. Diese Entleerung lässt sich durch entsprechende Querschnittswahl der Belüftungsöffnung so einstellen, dass die Belüftung nicht plötzlich erfolgt, sondern allmählich, so dass die beschriebene Belästigung oder Gefährdung des Benutzers entfällt.

Besonders vorteilhaft ist es, wenn die Druckgaspatrone durch eine Haltevorrichtung im Anschlussschacht gehalten ist, die bei einem Herausziehen der Druckgaspatrone aus dem Anschlussschacht die Druckgaspatrone erst völlig freigibt, wenn das Dichtstück die Belüftungsstellung erreicht oder in Richtung auf die Ruhestellung überschritten hat. Dadurch ist sichergestellt, dass eine Entlüftung über die Belüftungsöffnung bereits zu einem Zeitpunkt erfolgt, in dem die Haltevorrichtung die Druckgaspatrone noch nicht völlig freigegeben hat, das heißt umgekehrt, dass bei der völligen Freigabe die Belüftung bereits erfolgt ist, so dass der Benutzer die Druckgaspatrone erst aus dem Anschlussschacht vollständig entnehmen kann, wenn die Druckgaspatrone schon entleert ist.

Es hat sich als günstig herausgestellt, wenn zur Abdichtung des Dichtstückes gegenüber der Innenwand des Anschlussschachtes in einer Umfangsnut der Innenwand eine an der Außenseite des Dichtstückes anliegende Ringdichtung angeordnet ist. Eine solche Ringdichtung in der Innenwand des Anschlussschachtes erfährt auch bei hoher Strömungsgeschwindigkeit des Druckgases in dem sich öffnenden Spalt zwischen Dichtstück und Anschlussschacht eine gleichmäßige Belastung, so dass die Gefahr reduziert wird, dass diese Ringdichtung ungewollt in diesen Spalt hineingezogen und beschädigt wird.

Es ist günstig, wenn das Dichtstück einen Abschnitt mit reduziertem Außendurchmesser aufweist, der beim Erreichen der Belüftungsstellung in den Bereich der Ringdichtung gelangt und dadurch einen Strömungsweg von dem stromabwärts des Dichtstückes gelegenen Abschnitt des Anschlussschachtes zu der Belüftungsöffnung freigibt. Während also in der Dichtstellung der normale, größere Außendurchmesser der Außenfläche des Dichtstückes an der Ringdichtung dichtend anliegt, ergibt sich die Öffnung eines Strömungsweges zur Belüftungsöffnung, sobald der Abschnitt mit reduziertem Außendurchmesser der Außenfläche in den Bereich der Ringdichtung gelangt.

Dabei kann es vorteilhaft sein, wenn das Dichtstück in dem Abschnitt mit reduziertem Durchmesser mindestens eine Durchbrechung aufweist, die den Zwischenraum zwischen der Außenwand des Dichtstückes und der Innenwand des Anschlussschachtes mit dem stromabwärts des Dichtstückes gelegenen Abschnitt des Anschlussschachtes verbindet. Durch diese Durchbrechung oder gegebenenfalls durch diese Durchbrechungen wird in dem Zwischenraum zwischen der Außenwand des Dichtstückes und der Innenwand des Anschlussschachtes ein Nebenweg für die Strömung eröffnet, so dass bei der Öffnung der Gasstrom nicht ausschließlich durch diesen Zwischenraum verläuft, sondern sowohl durch diesen Zwischenraum als auch durch die Durchbrechung. Dadurch wird die Ringdichtung in der Innenwand des Anschlussschachtes entlastet und es wird sichergestellt, dass diese nicht aufgrund der starken Strömung allein durch den Zwischenraum in diesen hineingesaugt und beschädigt wird und auch dabei den Abströmpfad in den Zwischenraum verschließt.

Bei einer besonders günstigen Ausführungsform ist vorgesehen, dass das Dichtstück hülsenförmig ausgebildet ist und sein Innenraum mit dem stromabwärts des Dichtstückes gelegenen Abschnitt des Anschlussschachtes in Verbindung steht.
Die Durchbrechung kann als radiale Bohrung in der Wand des hülsenförmigen Dichtstückes ausgebildet sein.

Bei einer abgewandelten Ausführungsform ist vorgesehen, dass zur Abdichtung des Dichtstückes gegenüber der Innenwand des Anschlussschachtes in einer Umfangsnut des Dichtstückes eine an der Innenwand des Anschlussschachtes anliegende Ringdichtung angeordnet ist. Hier ist also eine umgekehrte Anordnung der Ringdichtung und der Umfangsnut gewählt.

Es ist dabei besonders vorteilhaft, wenn in der Innenwand des Anschlussschachtes eine Erweiterung angeordnet ist, die der Ringdichtung gegenüberliegt, wenn das Dichtstück in der Belüftungsstellung steht, und die dabei einen Strömungsweg von dem stromabwärts des Dichtstückes gelegenen Abschnitt des Anschlussschachtes zu der Belüftungsöffnung freigibt. Während also in der Dichtstellung der normale, kleinere Innendurchmesser der Innenwand des Anschlussschachtes an der Ringdichtung dichtend anliegt, ergibt sich die Öffnung eines Strömungsweges zur Belüftungsöffnung, sobald die Erweiterung in der Innenwand des Anschlussschachtes in den Bereich der Ringdichtung gelangt, dieser Strömungsweg führt dann durch die Erweiterung hindurch an der Ringdichtung vorbei.

Bei einer besonders bevorzugten Ausführungsform ist vorgesehen, dass die Umfangsnut mindestens eine seitliche Ausnehmung aufweist, die die Umfangsnut auch dann mit der Belüftungsöffnung verbindet, wenn in der Belüftungsstellung des Dichtstückes die Ringdichtung nach außen in die Erweiterung hineingedrückt wird und damit mit Ausnahme der mindestens einen Ausnehmung der Strömungsweg von dem stromabwärts des Dichtstückes gelegenen Abschnitt des Anschlussschachtes zu der Belüftungsöffnung verschlossen wird. Selbst in diesem Fall also, in dem die Ringdichtung aufgeweitet wird und den Spalt zwischen der Erweiterung einerseits und dem Dichtstück andererseits in Richtung auf die Belüftungsöffnung verschließt, bleibt ein offener Strömungsweg erhalten, der durch die Ausnehmung oder die Ausnehmungen führt, die neben der Ringdichtung die die Ringdichtung aufnehmende Umfangsnut mit der Belüftungsöffnung verbinden.

Es ist weiterhin vorteilhaft, wenn zur Abdichtung des Dichtstückes gegenüber dem Anschlussschacht stromaufwärts der Ringdichtung eine in einer Umfangsnut angeordnete weitere Ringdichtung vorgesehen ist. Dabei kann die Umfangsnut in der Innenwand des Anschlussschachtes angeordnet sein und die weitere Ringdichtung stützt sich dann an der Außenfläche des Dichtstückes ab, es kann aber auch umgekehrt die Umfangsnut im Dichtstück angeordnet werden, so dass sich die weitere Ringdichtung an der Innenwand des Anschlussschachtes dichtend anlegt. Dadurch wird durch die beiden Ringdichtungen zwischen Dichtstück und Anschlussschacht eine besonders zuverlässige Abdichtung erzielt, die einmal sicherstellt, dass im normalen Betrieb kein Druckgas von dem stromabwärts des Dichtstückes gelegenen Abschnitt des Anschlussschachtes in den stromaufwärts des Dichtstückes gelegenen Abschnitt des Anschlussschachtes gelangt, die aber auch insbesondere dafür sorgt, dass beim Belüften die Gasfüllung ausschließlich durch die Belüftungsöffnung abströmt und nicht unkontrolliert zwischen Anschlussschacht und Dichtstück austritt.

Die Belüftungsöffnung kann insbesondere als Umfangsnut in der Innenwand des Anschlussschachtes ausgebildet sein.

Es ist vorteilhaft, wenn die Belüftungsöffnung als Drossel ausgebildet ist. Dies kann beispielsweise dadurch erfolgen, dass eine als Umfangsnut ausgebildete Belüftungsöffnung über eine Belüftungsöffnung mit engem Querschnitt mit dem Außenraum in Verbindung steht, so dass dadurch die Strömungsgeschwindigkeit der austretenden Gasfüllung begrenzt wird, die Gasfüllung also nicht schlagartig entweichen kann.

Es ist vorteilhaft, wenn das Dichtstück von einer Feder in Richtung auf die Ruhestellung belastet ist.

Bei einer besonders bevorzugten Ausführungsform ist vorgesehen, dass das Dichtstück aus einem hülsenförmigen Außenteil und einem die Dichtfläche für die Druckgaspatrone tragenden Innenteil gebildet wird und sich das Außenteil abgedichtet auf dem Innenteil abstützt.

Es ist weiterhin vorteilhaft, wenn die Druckgaspatrone in ihrem in den Anschlussschacht eingeschobenen Anschlussbereich mindestens einen Belüftungskanal aufweist, der den stromaufwärts des Dichtstückes und außerhalb der Dichtstelle zwischen Dichtstück und Druckgaspatrone gelegenen Abschnitt des Anschlussschachtes zur Umgebung hin öffnet. Dadurch wird dieser Abschnitt des Anschlussschachtes zuverlässig belüftet, so dass auch Gasmengen, die an der Dichtstelle zwischen dem Dichtstück einerseits und der Druckgaspatrone andererseits ungewollt entweichen, nicht in diesem Abschnitt des Anschlussschachtes gesammelt werden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Längsschnittansicht eines Anschlusses ei- ner Druckgaspatrone mit eingesetzter Druckgaspatrone;
- Figur 2:: eine Teilansicht des Anschlussteils der Figur 1 in Richtung des Pfeiles A in Figur 1;
- Figur 3:: eine Detailansicht entsprechend dem Ausschnitt B in Figur 2 mit dem Regelventil in Öffnungsstellung;
- Figur 4:: eine Ansicht ähnlich Figur 3 mit dem Regelventil in Schließstel- lung;
- Figur 5:: eine Ansicht ähnlich Figur 3 mit dem Anschlusselement in Ent- lastungsstellung;
- Figur 6:: eine Ansicht ähnlich Figur 3 ohne instrumentenseitigen An- schlussstutzen am Ausströmende des Anschlusses und mit teil- weise aus dem Anschlussschacht herausgezogener Druckgas- patrone;
- Figur 7:: eine Ansicht des Ausschnittes C in Figur 6 in vergrößerter Dar- stellung;
- Figur 8:: eine Ansicht ähnlich Figur 7 bei einem abgewandelten Ausfüh- rungsbeispiel mit einer Ringdichtung im Dichtstück und einer Erweiterung in der Innenwand des Anschlussschachtes;
- Figur 9:: eine Ansicht ähnlich Figur 8 mit einer aufgeweiteten, in die Er- weiterung in der Innenwand des Anschlussschachtes eintreten- der Ringdichtung;
- Figur 10:: eine Draufsicht auf das Dichtstück der Figur 9 im Bereich der Ringdichtung mit einer seitlichen Ausnehmung der die Ringdich- tung aufnehmenden Umfangsnut;
- Figur 11:: eine vergrößerte Schnittansicht des ausströmseitigen Endes des Anschlusses mit dem Schließkörper in Schließposition;
- Figur 12:: eine Schnittansicht der Druckgaspatrone mit der Druckgaspat- rone in der richtigen Einbaurichtung und

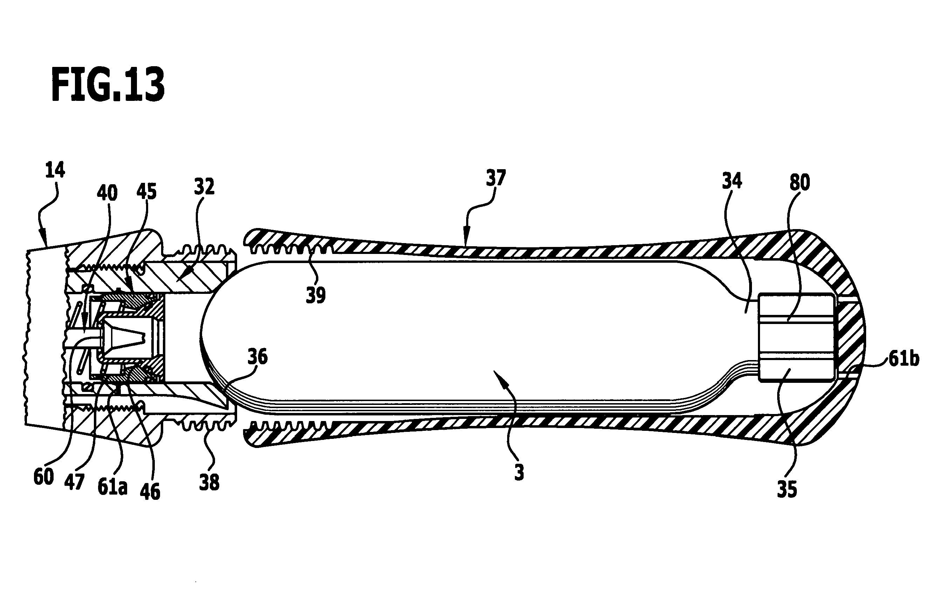

- Figur 13:: eine Schnittansicht ähnlich Figur 9 mit der Druckgaspatrone in falscher Einbaurichtung.

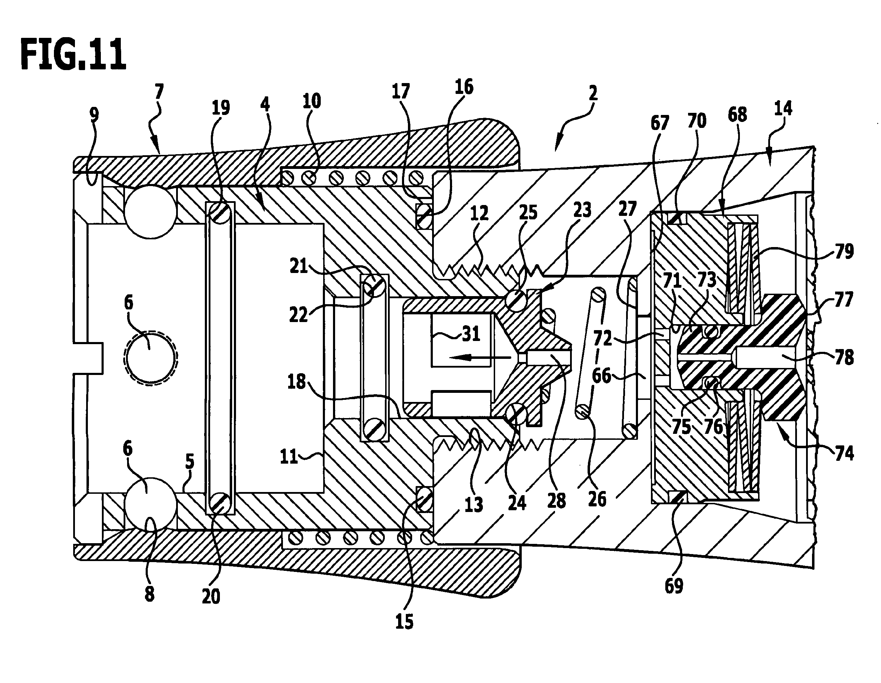

In der Zeichnung ist eine Druckgasversorgung für ein Instrument dargestellt, insbesondere ein medizinisches Instrument, beispielsweise ein Instrument zum Setzen von Heftklammern, zum Antrieb eines Schneidgerätes oder dergleichen. Von diesem Instrument ist jedoch in der Zeichnung nur ein rohrförmiger Anschlussstutzen 1 dargestellt, der über einen Anschluss 2 mit einer Druckgaspatrone 3 verbunden wird. Nachfolgend wird dieser Anschluss näher beschrieben, es versteht sich aber, dass dieser Anschluss über einen Anschlussstutzen 1 mit einem beliebigen druckgasbetriebenen Instrument verbunden werden kann. Zu diesem Zweck weist der Anschluss eine Einsteckbuchse 4 auf mit einer zylindrischen Einsteckkammer 5, in diese Einsteckkammer 5 kann von einer Seite der ebenfalls zylindrische Anschlussstutzen 1 eingeschoben werden und dort mit Hilfe von in der Einsteckbuchse 4 radial verschieblich gelagerten kugelförmigen Sperrelementen 6 in axialer Richtung fixiert werden. Diese Sperrelemente 6 können zwischen einer radial eingeschobenen Stellung, in der sie in entsprechende Vertiefungen des Anschlussstutzens 1 eintauchen, und einer radial ausgeschobenen Freigabestellung verschoben werden, in der sie so weit nach außen verlagert sind, dass der Anschlussstutzen 1 frei in die Einsteckkammer 5 eingeschoben und wieder aus dieser herausgezogen werden kann. Zur Festlegung der kugelförmigen Sperrelemente 6 in der eingeschobenen Sperrstellung dient eine die Einsteckbuchse 4 umgebende und gegenüber dieser axial verschiebliche Hülse 7 mit zwei nebeneinander angeordneten Abschnitten 8, 9, die jeweils unterschiedliche Innendurchmesser aufweisen. Bei Anlage des Abschnittes 8 mit kleinem Innendurchmesser werden die kugelförmigen Sperrelemente 6 radial nach innen geschoben, bei Anlage des Abschnittes 9 mit dem größeren Innendurchmesser können die kugelförmigen Sperrelemente 6 radial nach außen verschoben werden. Die Hülse 7 wird durch eine die Einsteckbuchse 4 umgebende Schraubenfeder 10 so verschoben, dass der Abschnitt 8 mit kleinerem Innendurchmesser die kugelförmigen Sperrelemente 6 überdeckt. Um diese kugelförmigen Sperrelemente 6 freigeben zu können, muss die Hülse 7 entgegen der Wirkung der Schraubenfeder 10 radial zurückgeschoben werden (Figur 11).

An ihrem dem offenen Ende der Einsteckkammer 5 gegenüberliegenden Ende trägt die Einsteckbuchse 4 einen zentralen Außengewindestutzen 12, mit dem die Einsteckbuchse 4 in eine Innengewindebohrung 13 eines zentralen Anschlussstückes 14 eingeschraubt ist. Dabei wird die Einsteckbuchse 4 gegenüber dem zentralen Anschlussstück 14 durch eine Ringdichtung 15 abgedichtet, die in einer Ringnut 16 an der Rückseite der Einsteckbuchse 4 angeordnet ist und sich an der Stirnseite 17 des zentralen Anschlussstückes 14 anlegt.

Die Einsteckkammer 5 geht unter Ausbildung einer Stufe 11 über in einen zentralen Strömungskanal 18, der den Außengewindestutzen 12 zentral durchsetzt. In eine Umfangsnut 19 an der Innenwand der Einsteckkammer 5 ist eine Ringdichtung 20 eingelegt, eine weitere Ringdichtung 21 ist in eine Umfangsnut 22 in der Innenwand des zentralen Strömungskanals 18 eingelegt.

In den zentralen Strömungskanal 18 ist in Längsrichtung des Strömungskanals 18 verschiebbar ein Schließkörper 23 eingesteckt, der teilweise aus dem Außengewindestutzen 12 hervorsteht und dort einen Außendurchmesser aufweist, der größer ist als der Innendurchmesser des zentralen Strömungskanals 18. In diesem Bereich ist in eine Umfangsnut 24 des Schließkörpers 23 eine Ringdichtung 25 eingelegt, die sich an die Außenkante des zentralen Strömungskanals 18 anlegt, wenn man den Schließkörper in Richtung auf die Einsteckkammer 5 verschiebt. In dieser Stellung verschließt der Schließkörper 23 damit den Strömungskanal 18, diese Stellung wird als Schließposition bezeichnet. In diese Position wird der Schließkörper durch eine Schraubenfeder 26 gedrückt, die sich einerseits an der Außenseite des Schließkörpers 23 und andererseits an einer Stufe 27 des Anschlussstückes 14 abstützt.

In dem Schließkörper 23 ist ein Durchlass 28 in Form einer Bohrung mit sehr kleinem Durchmesser angeordnet. Dieser Durchlass 28 verbindet den Innenraum des Anschlussstückes 14 damit mit dem zentralen Strömungskanal 18. Der Durchmesser ist jedoch so gering, dass pro Zeiteinheit nur eine sehr geringe Gasmenge hindurchtreten kann, in der Zeichnung ist der Durchmesser dieses Durchlasses 28 zur Verdeutlichung relativ groß ausgebildet. Tatsächlich kann es sich dabei um eine ganz dünne Bohrung handeln oder auch um einen porösen Wandabschnitt an der Rückseite des Schließkörpers, der einen langsamen Abbau eines Gasüberdruckes aus dem Inneren des Anschlussstückes 14 in Richtung auf die offene Einsteckkammer 5 hin zulässt.

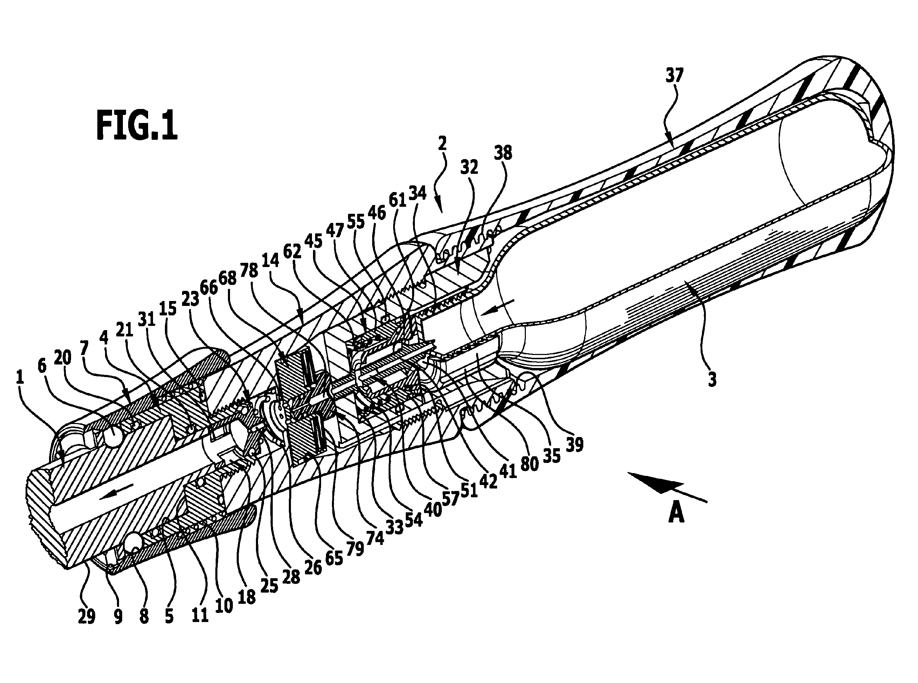

Der Anschlussstutzen 1 eines Instrumentes weist einen ersten Abschnitt 29 mit einem Außendurchmesser auf, der dem Innendurchmesser der Einsteckbuchse 4 entspricht, und einen zweiten Abschnitt 30 mit einem wesentlich geringeren Außendurchmesser, der dem Innendurchmesser des zentralen Strömungskanals 18 entspricht (Figur 1). Beim Einsetzen des Anschlussstutzens 1 in die Einsteckbuchse 4 wird der Abschnitt 29 in der Einsteckbuchse 4 aufgenommen, der Abschnitt 30 dagegen in dem zentralen Strömungskanal 18. Dabei schiebt der Abschnitt 30 des Anschlussstutzens 1 den Schließkörper 23 entgegen der Wirkung der Schraubenfeder 26 aus dem zentralen Strömungskanal 18 heraus in eine Öffnungsposition, in der die Ringdichtung 25 von der Kante des zentralen Strömungskanals 18 abgehoben ist, so dass die Schließwirkung in diesem Bereich aufgehoben ist. In der Seitenwand des Schließkörpers 23 sind Fensteröffnungen 31 angeordnet, die eine Verbindung zwischen dem Innenraum des Anschlussstutzens 1 einerseits und dem Innenraum des Anschlussstückes 14 andererseits herstellen, sobald der Schließkörper 23 in der Öffnungsposition steht. In dieser Position wird also ein Gasstrom aus dem Innenraum des Anschlussstückes 14 in den Innenraum des Anschlussstutzens 1 nicht behindert.

Eine Behinderung tritt erst auf, wenn der Schließkörper wieder in der Schließposition steht, und dies setzt voraus, dass der Anschlussstutzen 1 aus der Einsteckbuchse 4 entfernt ist, wie dies in Figur 11 dargestellt ist. Nur in dieser Stellung wird der Durchlass 28 überhaupt wirksam. In der Öffnungsstellung ist der Querschnitt des Durchlasses 28 gegenüber den übrigen Strömungsquerschnitten für den Gasstrom so klein, dass er praktisch nicht ins Gewicht fällt. In der Schließposition dagegen erfolgt infolge des Durchlasses 28 kein hermetisches Verschließen, sondern der Druck im Inneren des Anschlussstückes 14 kann sich über diesen Durchlass 28 allmählich abbauen, wobei die Dauer dieses Abbaus vom Querschnitt des Durchlasses 28 abhängt.

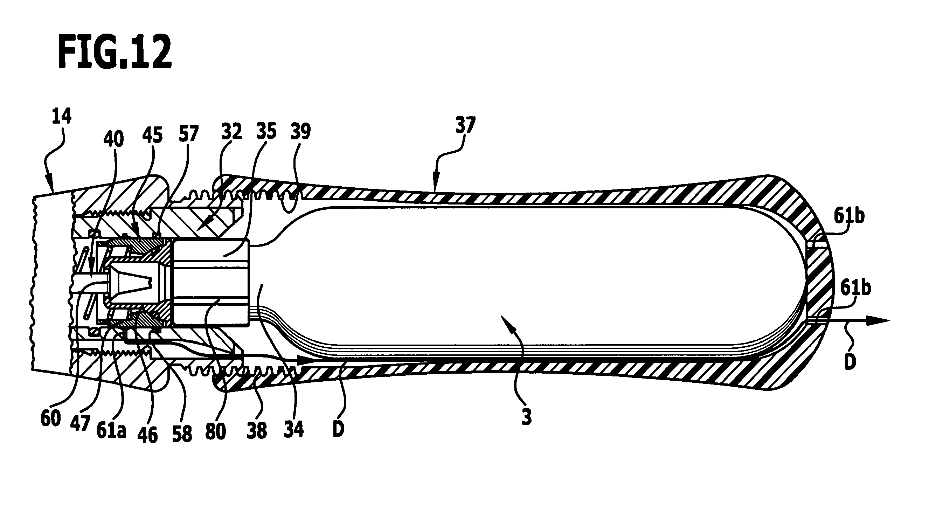

Auf der der Einsteckbuchse 4 gegenüberliegenden Seite des Anschlussstückes 14 ist in dieses ein topfförmiger Aufnahmekörper 32 eingeschraubt. Am hinteren Ende des Anschlussstückes 14 bildet ein offener Innenraum des Aufnahmekörpers 32 einen Anschlussschacht 33 für den zylindrischen Anschlussbereich 34 der Druckgaspatrone 3. Diese zylindrische Druckgaspatrone 3 ist am rückwärtigen Ende kugelförmig abgerundet, am vorderen Ende läuft sie flaschenhalsähnlich aus in einem zylindrischen Anschlussbereich 34, der von einer Dichtkappe 35 aus einem verformbaren Kunststoffmaterial überfangen ist. Die Druckgaspatrone 3 kann mit dem Anschlussbereich 34 in den Anschlussschacht 33 eingeschoben werden, die Einschubtiefe wird begrenzt dadurch, dass der Bereich der Druckgaspatrone 3 mit größerem Außendurchmesser am entsprechend geformten Rand 36 des Aufnahmekörpers 32 anschlägt. Dieser Rand 36 verhindert im übrigen auch, dass die Druckgaspatrone 3 verkehrt in den Anschlussschacht 33 eingesetzt wird, also mit dem kugelförmigen Ende voran, wie dies in Figur 12 dargestellt ist. In diesem Falle schlägt das kugelförmige Ende an dem Rand 36 an und verhindert somit eine weitere Annäherung der Druckgaspatrone an das Anschlussstück 14 und damit auch eine Beschädigung des Anstechdornes 40 (siehe unten).

Die Druckgaspatrone 3 kann mittels einer Überwurfhülse 37 an dem Anschlussstück 14 festgelegt werden. Dazu trägt das Anschlussstück 14 an seinem rückwärtigen Ende ein Außengewinde 38, auf welches die Überwurfhülse 37 mit einem Innengewinde 39 aufgeschraubt werden kann. Die Dimensionierung ist dabei so gewählt, dass die Überwurfhülse 37 die Druckgaspatrone 3 bei korrekter Orientierung so weit gegen das Anschlussstück 14 verschiebt, bis der Bereich mit größerem Außendurchmesser der Druckgaspatrone 3 an dem Rand 36 des Aufnahmekörpers 32 anliegt. Ist die Druckgaspatrone falsch orientiert, steht sie so weit von dem Anschlussstück 14 vor, dass die über die falsch orientierte Druckgaspatrone 3 geschobene Überwurfhülse mit ihrem Innengewinde 39 das Außengewinde 38 nicht erreichen kann. Ein Aufschrauben der Überwurfhülse 37 ist damit nicht möglich. Dies dient der Kontrolle für die korrekte Orientierung der Druckgaspatrone 3 relativ zu dem Anschlussstück 14.

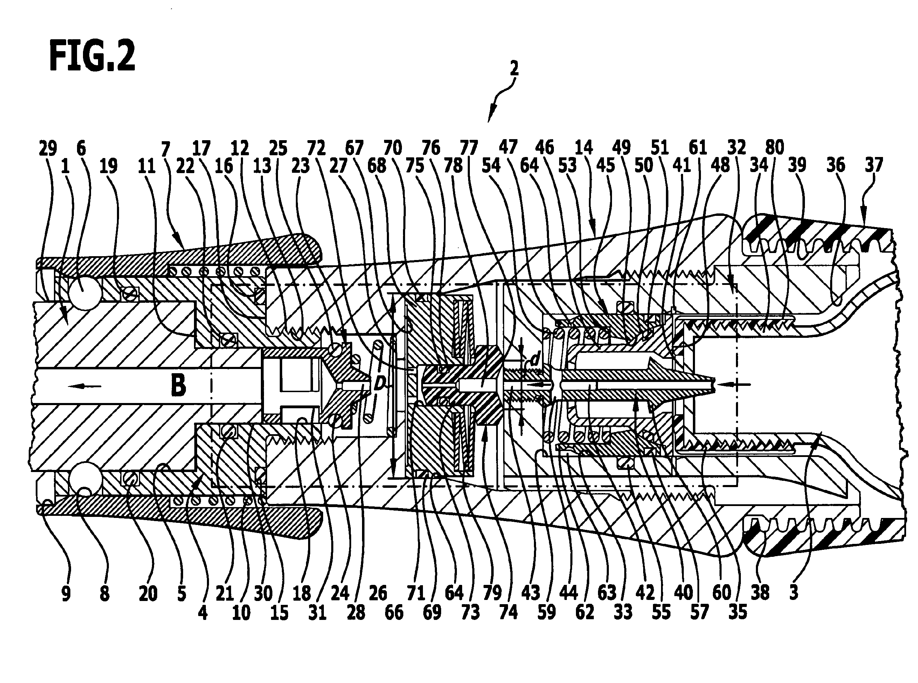

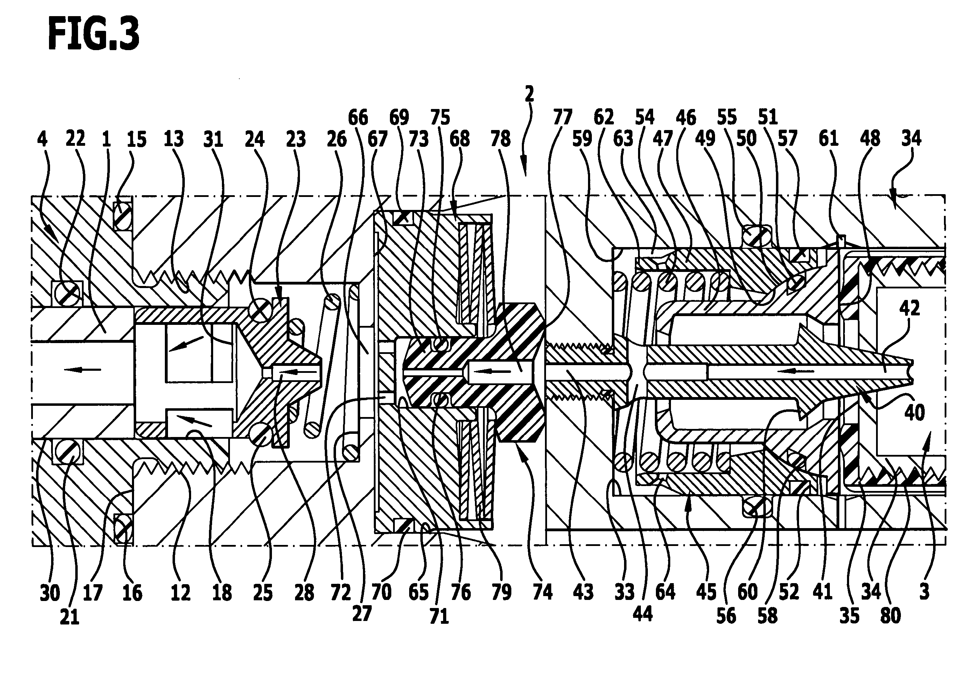

In dem Anschlussschacht 33 ist ein zentraler Anstechdorn 40 angeordnet, der gegenüber dem Aufnahmekörper 32 in axialer Richtung unverschieblich gelagert ist, beispielsweise ist er in den Boden des Anschlussschachtes eingeschraubt. Dieser Anstechdorn 40 steht vom Boden des Anschlussschachtes 33 so weit hervor, dass er die Druckgaspatrone 3 an ihrer Stirnfläche 41 durchbohrt, wenn die Druckgaspatrone 3 vollständig in den Anschlussschacht 33 eingeschoben ist (Figuren 2 und 3).

Der Anstechdorn 40 wird von einem rohrförmigen Kanal 42 durchsetzt, der ein Teil des gesamten Strömungskanals vom Inneren der Druckgaspatrone 3 bis zu dem Anschlussstutzen 1 ist, also ein Teil eines das Anschlussstück 14 durchsetzenden Zufuhrkanals 43 für das Druckgas. Der Kanal 42 in dem Anstechdorn 40 steht außerdem über eine Querbohrung 44 mit dem Innenraum des Anschlussschachtes 33 in Verbindung.

In dem Anschlussschacht 33 ist den Ansteckdorn 40 im Abstand umgebend ein im Anschlussschacht 33 in dessen Längsrichtung verschieblich gelagertes Dichtstück 45 angeordnet, welches aus zwei Teilen besteht, nämlich einem hutförmigen Innenteil 46 und einem hülsenförmigen Außenteil 47. Das Innenteil 46 weist an seinem der Druckgaspatrone 3 zugewandten Ende eine stirnseitige Dichtfläche 48 auf, die sich bei eingeschobener Druckgaspatrone 3 an deren Dichtkappe 35 dichtend anlegt, diese Dichtkappe 35 kann zur Verbesserung der Dichtwirkung im Dichtbereich wulstförmig verbreitert sein.

Der hülsenförmige Außenteil 47 stützt sich mit einer kugelkalottenförmigen Innenfläche 49 an einer ebenfalls kugelkalottenförmigen Außenfläche 50 des Innenteils 46 ab, in diesem Bereich sind Innenteil 46 und Außenteil 47 durch eine Ringdichtung 51 gegeneinander abgedichtet, die in eine Umfangsnut 52 in der Außenfläche 50 eingelegt ist. An einer Stufe 53 des Außenteils 47 stützt sich eine den Anstechdorn 40 im Abstand umgebende Schraubenfeder 54 ab, deren anderes Ende am Boden des Aufnahmeschachtes 33 anliegt und somit das Außenteil 47 in Richtung auf die Druckgaspatrone 3 belastet. Dadurch wird auch das Innenteil 46 in derselben Richtung verschoben, da das Außenteil 47 über seine Innenfläche 49 an der Außenfläche 50 des Innenteils 46 anliegt. Die Schraubenfeder 54 schiebt damit das gesamte Dichtstück 45 gegen die Stirnfläche 41 der in den Anschlussschacht 33 eingesetzten Druckgaspatrone 3 und drückt dabei die Dichtfläche 48 gegen die Dichtkappe 35, so dass in diesem Bereich eine Abdichtung erfolgt.

Das Außenteil 47 des Dichtstückes 45 ist gegenüber der Innenwand des Anschlussschachtes 33 ebenfalls abgedichtet, und zwar einmal durch eine Ringdichtung 55, die in eine Umfangsnut 56 in der Innenwand des Anschlussschachtes 33 eingelegt ist und an der Außenfläche des Außenteiles 47 anliegt, und zum anderen durch eine Ringdichtung 57, die in eine Umfangsnut 58 in der Außenfläche des Außenteils 47 eingelegt ist und an der Innenwand des Anschlussschachtes 33 anliegt. Die Abdichtung in diesem Bereich könnte auch durch eine Ringdichtung erfolgen, die in eine Umfangsnut in der Innenwand des Anschlussschachtes 33 eingearbeitet ist, so dass die Ringdichtung dann dichtend an die Außenfläche des Außenteils 47 angelegt wäre, wie dies in Figur 12 dargestellt ist.

Durch die beschriebene Abdichtung wird das Dichtstück 45 gegenüber der Innenwand des Anschlussschachtes 33 abgedichtet, es ergibt sich damit ein abgedichteter Abschnitt 59 des Anschlussschachtes 33, der von der Druckgaspatrone 3 aus gesehen stromabwärts des Dichtstückes 45 angeordnet ist.

Dieses Dichtstück 45 steht vor dem Einschieben der Druckgaspatrone 3 in den Anschlussschacht 33 in einer Ruhestellung, bei der die Schraubenfeder 54 zumindest teilweise entspannt ist, dabei legt sich das hutförmige Innenteil 46 an einen Vorsprung 60 auf der Außenseite des Anstechdornes 40 an, der damit eine Verschiebung des Dichtstückes 45 begrenzt. Beim Einschieben einer Druckgaspatrone 3 schiebt diese dann das Dichtstück 45 aus dieser Ruhestellung entgegen der Wirkung der Schraubenfeder 54 in die Dichtstellung, in der die Schraubenfeder 54 das Dichtstück 45 gegen die Dichtkappe 35 der Druckgaspatrone 3 drückt.

Die von der Dichtung zwischen der Dichtkappe 35 und der Dichtfläche 48 eingeschlossene Querschnittsfläche des Dichtstückes 45 ist kleiner als die von den Ringdichtungen 55 und 57 eingeschlossene Querschnittsfläche, so dass aufgrund des Druckaufbaues in dem vom Dichtstück 45 abgeschlossenen Abschnitt 59 des Anschlussschachtes 33 eine Differenzkraft auf das Dichtstück 45 ausgeübt wird, welches die Wirkung der Schraubenfeder 54 verstärkt. Diese Differenzkraft nimmt mit zunehmendem Druck in dem Abschnitt 59 zu. Es handelt sich somit um eine selbstverstärkende Wirkung des Dichtstückes 45.

In der Innenwand des Anschlussschachtes 33 ist eine Umfangsnut 61 angeordnet, die außenseitig mit der Umgebung in Verbindung steht. Über diese Umfangsnut 61 kann also der Anschlussschacht 33 belüftet werden. Diese Umfangsnut 61 ist in Richtung auf das offene Ende des Anschlussschachtes 33 gegenüber der Ringdichtung 55 versetzt, so dass der Abschnitt 59 des Anschlussschachtes 33 gegenüber dieser Umfangsnut 61 abgedichtet ist, solange das Dichtstück 45 in der Dichtstellung steht. Dann liegt nämlich die Ringdichtung 55 an der Außenfläche des Dichtstückes 45 dichtend an.

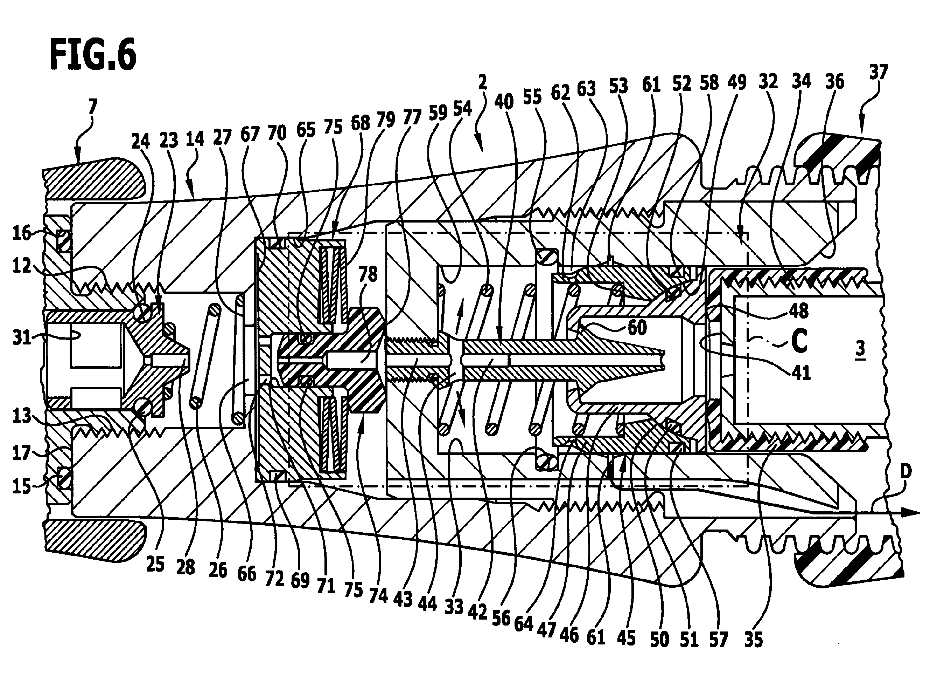

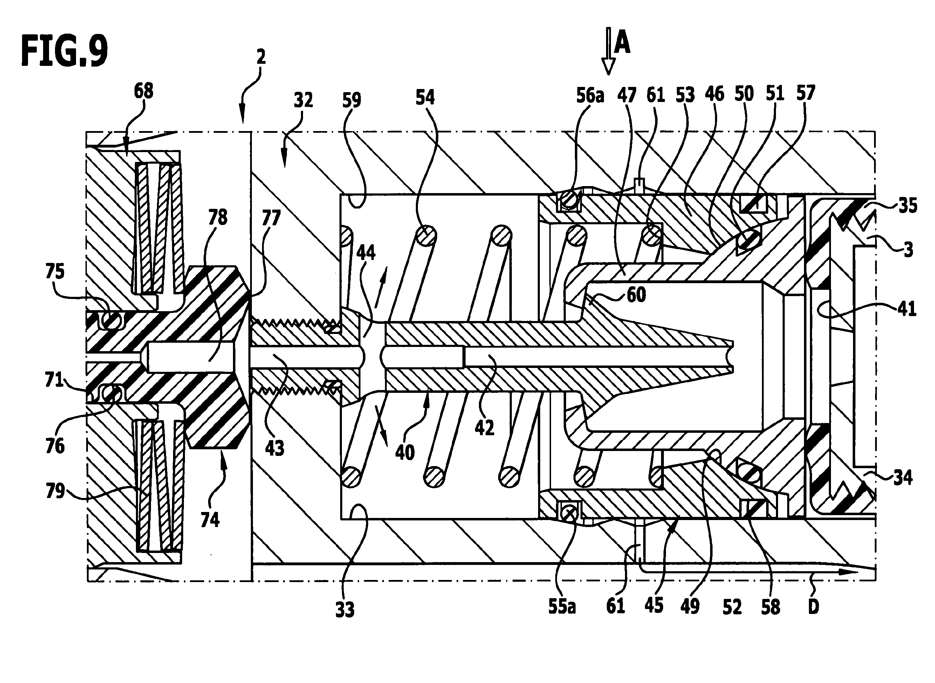

Die Umfangsnut 61 steht über eine Belüftungsbohrung 61a mit dem Innenraum in Verbindung, der einmal durch das Anschlussstück 14 und zum anderen durch die Überwurfhülse 37 umschlossen wird. Wie aus der Darstellung der Figur 12 deutlich wird, trägt die Überwurfhülse 37 an ihrem dem Innengewinde 39 abgewandten Ende mehrere Ausströmöffnungen 61b, durch die das durch die Umfangsnut 61 ausströmende Gas in die Umgebung abströmen kann. Das Ausströmen erfolgt dabei also in einem Bereich, der von den Funktionsteilen des Instrumentes maximal entfernt ist und bei dem keine Gefahr besteht, dass der Benutzer diesen Bereich ergreift, so dass auch keine Belästigung oder Gefährdung des Benutzers eintreten kann. Der Strömungsweg des über die Umfangsnut 61, die Belüftungsbohrung 61a, den Innenraum des Anschlussstückes 14 sowie der Überwurfhülse 37 und die Austrittsöffnungen 61b austretenden Gases ist in den Figuren 6 und 9 durch die Pfeile D angedeutet.

Die Belüftungsbohrung 61a hat einen sehr kleinen Querschnitt und wirkt somit als Drosselstelle zwischen der Umfangsnut 61 und dem Außenraum. Dadurch wird sichergestellt, dass die Strömungsgeschwindigkeit der austretenden Gasfüllung begrenzt wird, die Gasfüllung also nicht schlagartig entweicht.

Das Dichtstück 45 weist an seinem dem Boden des Anschlussschachtes 33 zugewandten Ende einen Randbereich 62 auf mit einem Außendurchmesser, der geringer ist als der Außendurchmesser der übrigen Außenfläche des Außenteils 47 des Dichtstückes 45. Sobald dieser Randbereich 62 in den Bereich der Ringdichtung 55 gelangt, wird daher die Dichtwirkung im Bereich der Ringdichtung 55 aufgehoben, das heißt es bildet sich an dieser Stelle ein schmaler Spalt 63 aus zwischen der Ringdichtung 55 und dem Randbereich 62 des Außenteils 47. Durch diesen Spalt 63 wird eine Verbindung zwischen dem Abschnitt 59 einerseits und der belüfteten Umfangsnut 61 andererseits hergestellt, und über diese Verbindung kann der Abschnitt 59 belüftet werden, das heißt ein in ihm aufgebauter Überdruck kann abgebaut werden.

Bei diesem Abbau können im Spalt 63 sehr hohe Strömungsgeschwindigkeiten entstehen, durch die die Gefahr besteht, dass die Ringdichtung 55 in den Spalt 63 hineingezogen und daher beschädigt wird, außerdem würde der Strömungspfad zwischen dem Abschnitt 59 und der Umfangsnut 61 dadurch verschlossen. Um dies zu verhindern, ist das hülsenförmige Außenteil 47 im Bereich des Randbereiches 62 mit radialen Entlastungsbohrungen 64 versehen, durch die Gas aus dem Abschnitt 59 in den Spalt 63 einströmen kann. Dadurch wird in diesem Bereich das Auftreten eines zu starken Unterdruckes verhindert und damit auch das ungewollte Einziehen der Ringdichtung 55 in den Spalt 63.

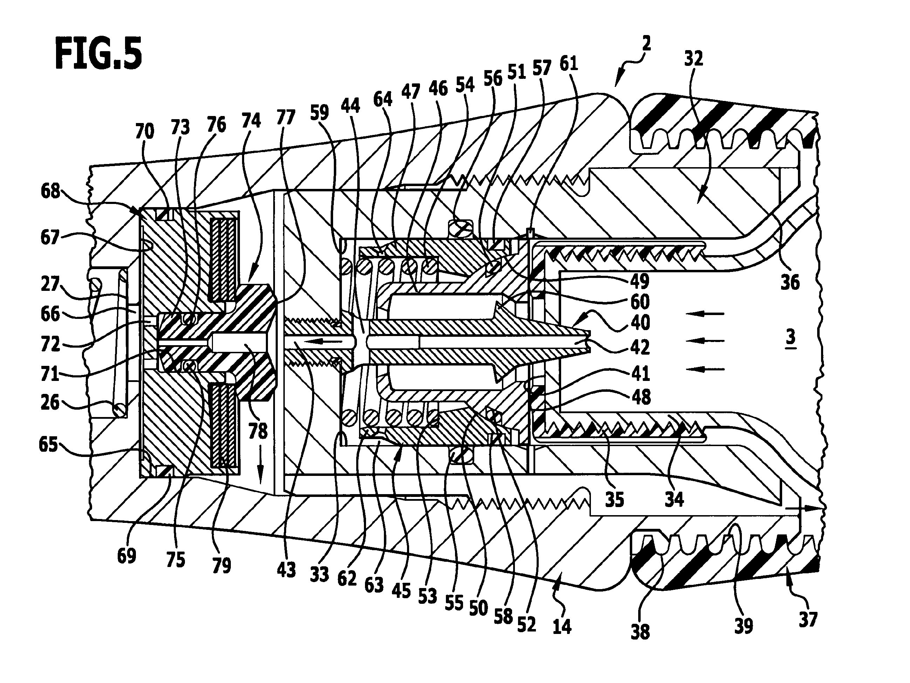

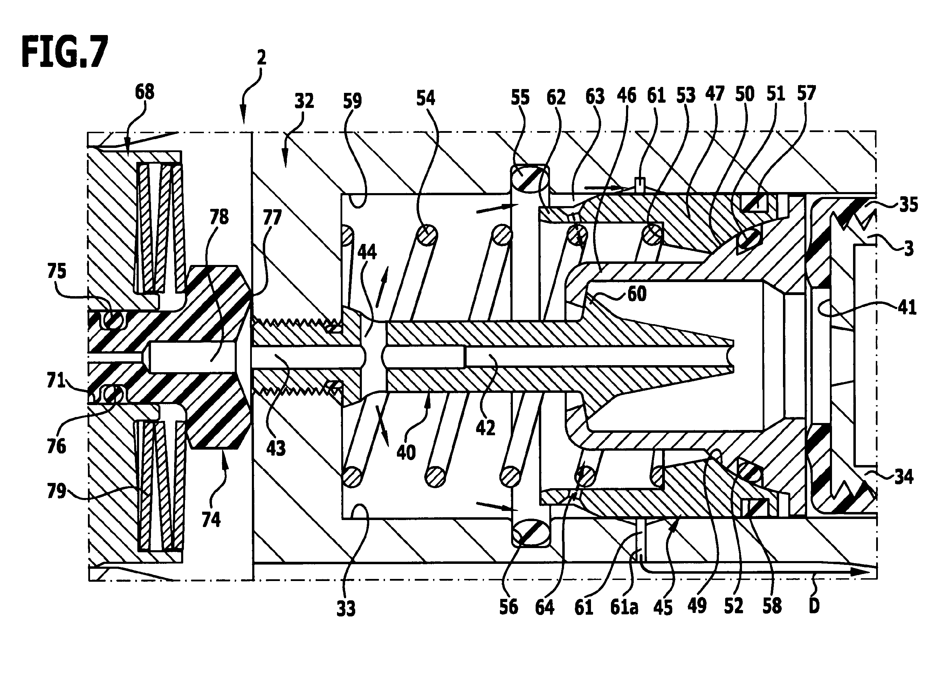

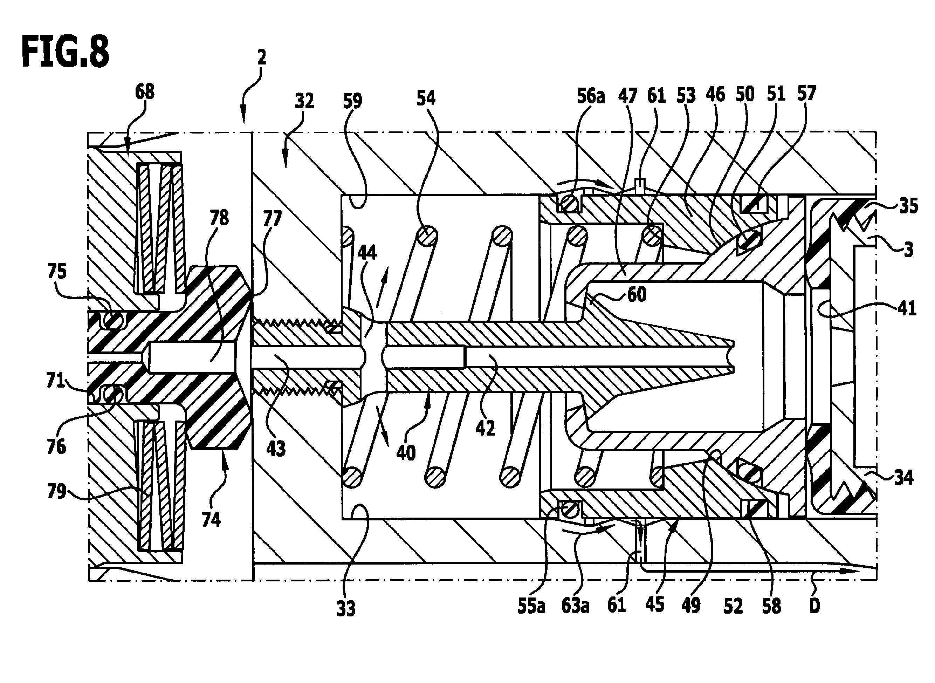

Während bei dem Ausführungsbeispiel der Figuren 1 bis 7 die Abdichtung zwischen Dichtstück 45 und der Innenwand des Anschlussschachtes 33 durch eine Ringdichtung 55 erfolgt, die in eine Umfangsnut 56 in der Innenwand des Anschlussschachtes 33 eingelegt ist, wird bei dem Ausführungsbeispiel der Figuren 7 bis 10, das im übrigen weitgehend gleich aufgebaut ist und bei dem gleiche Teile daher mit denselben Bezugszeichen versehen sind, eine Abdichtung des Dichtstückes 45 gegenüber der Innenwand des Anschlussschachtes 33 durch eine Ringdichtung 55a erreicht, die in eine Umfangsnut 56a in der Außenwand des Dichtstückes 45 eingelegt ist und die sich normalerweise an die Innenwand des Anschlussschachtes 33 dichtend anlegt.

Bei dieser Ausgestaltung ist in der Innenwand des Anschlussschachtes 33 eine umfangsnutförmige Erweiterung 63a angeordnet, und zwar derart, dass die Ringdichtung 55a dieser Erweiterung 63a gegenüberliegt, wenn sich das Dichtstück 45 in der Belüftungsstellung befindet. Durch die Erweiterung 63a ergibt sich dadurch ein Strömungsweg, der an der Ringdichtung 55a vorbeiführt und den stromabwärts des Dichtstückes 45 gelegenen Abschnitt 59 des Anschlussschachtes 33 verbindet mit der Umfangsnut 61 und der Belüftungsbohrung 61a. Dieses Auftreten eines Nebenstrompfades entspricht bei dem Ausführungsbeispiel der Figuren 1 bis 7 der Belüftungsstellung, bei der der Randbereich 62 mit dem kleineren Außendurchmesser der Ringdichtung 55 gegenübersteht.

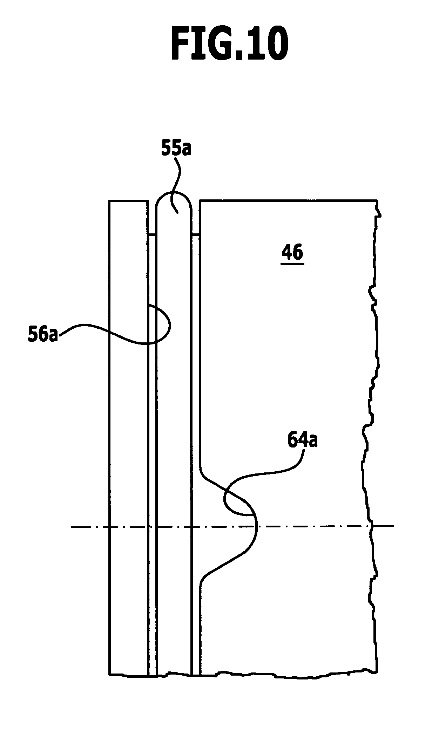

Bei dem in Figur 9 dargestellten Ausführungsbeispiel könnte die Ringdichtung 55a durch die an ihr vorbeiströmende Gasströmung mitgenommen und so aufgeweitet werden, dass sie nach außen in die Erweiterung 63a hineingedrückt wird und somit den Spalt zwischen dem Dichtstück 45 und der Innenwand des Anschlussschachtes 33 verschließt. Dann wäre eine Strömungsverbindung zu der Umfangsnut 61 und der Belüftungsbohrung 61a unterbunden. Um dies zu verhindern, ist bei dem Ausführungsbeispiel der Figuren 9 und 10 seitlich an der Umfangsnut 56a mindestens eine Ausnehmung 64a angeordnet, die im wesentlichen als Vertiefung der Seitenwand der Umfangsnut 56a ausgebildet ist und die sicherstellt, dass auch bei aufgeweiteter Ringdichtung 55a die Umfangsnut 56a mit der Umfangsnut 61 in Strömungsverbindung steht, wenn das Dichtstück 45 in der Belüftungsstellung steht. Auch wenn sich die Ringdichtung 55a aufweitet, kann sie nämlich die Ausnehmung 64a oder die Ausnehmungen 64a, die über den Umfang der Umfangsnut 56a verteilt sein können, nicht verschließen, so dass in diesem Bereich ein Strömungsweg frei bleibt.

Solange die Überwurfhülse 37 fest auf das Anschlussstück 14 aufgeschraubt ist und dadurch die Druckgaspatrone 3 vollständig in den Anschlussschacht 33 eingeschoben ist, bleibt das Dichtstück 45 in der Dichtstellung, die Umfangsnut 61 bleibt daher verschlossen. Sobald der Benutzer jedoch die Überwurfhülse 37 teilweise vom Anschlussstück 14 abschraubt, wird das Dichtstück 45 unter dem Einfluss der Schraubenfeder 54 und der vom Gasdruck hervorgerufenen Druckdifferenz in Richtung auf die Ruhestellung verschoben, da die Druckgaspatrone 3 nunmehr frei ist, zumindest teilweise aus dem Anschlussschacht 33 auszutreten. Dabei gelangt das Dichtstück zwischen der Dichtstellung und der Ruhestellung in eine dazwischen liegende Entlastungsstellung, in der der Randbereich 62 der Ringdichtung 55 gegenüberliegt und in der sich die Umfangsnut 61 zum Abschnitt 59 hin öffnet. Dies führt dazu, dass das Druckgas aus der Druckgaspatrone in die Umgebung entweichen kann, allerdings nicht schlagartig, sondern allmählich, da der Querschnitt der Umfangsnut 61 und der sich anschließenden Bohrung 61a klein ist. Es erfolgt also beim Abschrauben der Überwurfhülse 37 gleichzeitig mit diesem Abschraubvorgang eine Entleerung der Druckgaspatrone 3 ohne ein plötzliches Ausströmen des Druckgases und ohne das damit verbundene unangenehme Zischen des Druckgases. Durch die Abdichtung zwischen dem Dichtstück 45 und der Innenwand des Anschlussschachtes 33 durch die Ringdichtung 57 wird auch sichergestellt, dass die ausströmende Gasfüllung ausschließlich über die Umfangsnut 61 strömt und nicht unkontrolliert zwischen dem Dichtstück 45 und der Innenwand des Anschlussschachtes 33 austritt.

Ein vollständiges Abschrauben der Überwurfhülse 37 und damit eine vollständige Entnahme der Druckgaspatrone 3 ist erst nach einigen Umdrehungen der Überwurfhülse 37 möglich, und diese Zeit reicht aus, um die Druckgaspatrone 3 zumindest weitgehend zu entleeren, so dass bei der Entnahme der Druckgaspatrone 3 aus dem Anschlussschacht 33 diese ganz oder weitgehend entleert ist.

Zwischen dem Boden des Anschlussschachtes 33 einerseits und zwischen der Stufe 27 des Anschlussstückes 14 andererseits befindet sich im Anschlussstück 14 eine Ventilkammer 65, in die einerseits der Kanal 42 des Anstechdornes 40 einmündet und andererseits eine von der Stufe 27 umgebene Austrittsöffnung 66 austritt. Diese Austrittsöffnung 66 hat einen wesentlich kleineren Durchmesser als die Ventilkammer 65, so dass im Übergang von der Ventilkammer 65 zu der Austrittsöffnung 66 eine die Austrittsöffnung 66 umgebende Stufe 67 ausgebildet wird.

Im Inneren der Ventilkammer 65 ist ein scheibenförmiger Ventilkörper 68 verschieblich gelagert, der sich mittels einer ersten Ringdichtung 69, die in eine Umfangsnut 70 in der Außenwand des Ventilkörpers 68 eingelegt ist, gegenüber der Innenwand der Ventilkammer 65 abdichtet. In den Ventilkörper 68 ist auf dessen dem Aufnahmekörper 32 zugewandter Seite eine zentrale Lagerkammer 71 angeordnet in Form einer zylindrischen Sacklochbohrung, die an ihrem zentral verschlossenen Ende durch außermittig angeordnete Strömungsdurchlässe 72 mit dem stromabwärts des Ventilkörpers 68 angeordneten Abschnitt der Ventilkammer 65 und damit mit der Austrittsöffnung 66 verbunden ist.

In diese zylindrische Lagerkammer 71 ist der zylindrische Lagerstutzen 73 eines Anschlusselementes 74 eingeschoben und längsverschieblich in dieser Lagerkammer 71 gelagert. Eine zweite Ringdichtung 75, die in eine Umfangsnut 76 des Lagerstutzens 73 eingelegt ist, dichtet den Lagerstutzen 73 gegenüber der Innenwand der Lagerkammer 71 ab.

Das Anschlusselement 74 verbreitert sich außerhalb der Lagerkammer 71 und bildet in diesem Bereich eine ringförmige Dichtschulter 77 aus, die normalerweise dichtend an die Rückseite des Bodens des Aufnahmekörpers 32 angelegt ist und den Austritt des Anstechdorns 40 in die Ventilkammer 65 abdichtend umgibt. Das Anschlusselement 74 kann aus Kunststoffmaterial bestehen und insbesondere im Bereich der Dichtschulter 77 verformbar ausgebildet sein. Es wird von einem Strömungskanal 78 durchsetzt, der damit einerseits den Kanal 42 im Inneren des Anstechdorns 40 und andererseits die Lagerkammer 65 in dem unmittelbar an die Strömungsdurchlässe 72 anschließenden Bereich miteinander verbindet.

Der Durchmesser D der Ventilkammer 65 und des Ventilkörpers 68 ist im wesentlichen gleich groß, während der Durchmesser d der Dichtschulter 77 des Anschlusselementes 74 demgegenüber deutlich kleiner ist (Figur 2), beispielsweise gilt D = 19 mm und d = 6 mm, so dass das Verhältnis der wirksamen druckbeaufschlagten Flächen des Ventilkörpers 68 und des Anschlusselementes 74 bei etwa 10 liegt. Diese Verhältnisse können abweichen, beispielsweise kann das Verhältnis d:D zwischen 1:2 und 1:4 liegen.

An der dem Aufnahmekörper 32 zugewandten Seite stützt sich an dem Ventilkörper 68 eine das Anschlusselement 74 umgebende Tellerfeder 79 ab, die sich auf ihrer gegenüberliegenden Seite an dem verbreiterten Teil des Anschlusselementes 74 abstützt. Diese Tellerfeder 79 drückt damit den Ventilkörper 68 und das Anschlusselement 74 auseinander und drückt normalerweise den Ventilkörper 68 gegen die Stufe 67 und die Dichtschulter 77 des Anschlusselementes 74 gegen die Rückseite des Bodens des Anschlussschachtes 33 (Figur 3). In dieser Stellung kann durch den Anstechdorn 40 aus der Druckgaspatrone 3 austretendes Gas ungehindert durch das Anschlusselement 74 und den Ventilkörper 68 über die Strömungsdurchlässe 72 und die Austrittsöffnung 66 sowie den geöffneten Schließkörper 23 in den Anschlussstutzen 1 strömen. Der Schließkörper 23 steht dabei in seiner Öffnungsstellung, das Anschlusselement 74 in seiner Dichtstellung.

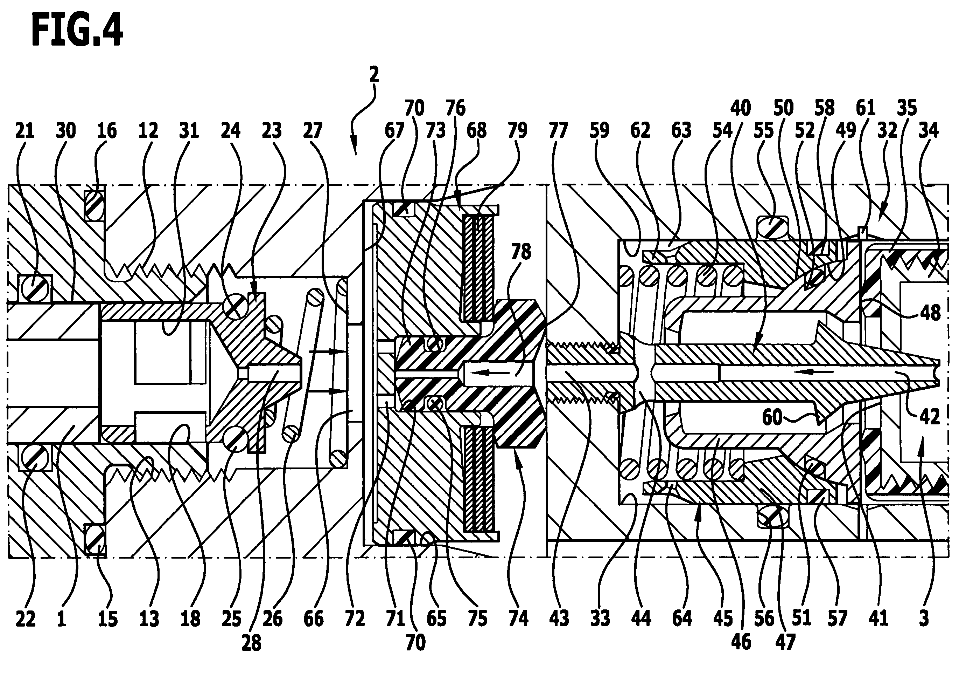

Wenn sich der Druck des ausströmenden Gases in der Lagerkammer 65 stromabwärts der ersten Ringdichtung 69 über einen bestimmten Wert erhöht, führt dies dazu, dass auf den Ventilkörper 68 eine Kraft wirkt, die der Federkraft der Tellerfeder 79 entgegengesetzt ist, da der stromaufwärts der ersten Ringdichtung 69 angeordnete Teil der Ventilkammer 65 nicht von dem Druckgas beaufschlagt ist. Dies führt beim Überschreiten eines bestimmten Gasdruckes dazu, dass der Ventilkörper 68 entgegen der Wirkung der Tellerfeder 70 gegen das Anschlusselement 74 verschoben wird, bis der verschlossene zentrale Bereich der Lagerkammer 71 sich abdichtend an die Stirnseite des Anschlusselementes 74 anlegt und dadurch den Strömungskanal 78 verschließt (Figur 4). Damit ist ein weiteres Nachströmen von Gas aus der Druckgaspatrone 3 beendet, eine Öffnung des Ventilkörpers 68 ergibt sich erst wieder, wenn der stromabwärts des Ventilkörpers 68 herrschende Druck abgebaut ist. Auf diese Weise wird der maximale Druck begrenzt, der in dem Bereich stromabwärts des Ventilkörpers 68 herrscht. Der Ventilkörper 68 wirkt als Druckbegrenzungsventil.

Im normalen Betrieb bleibt das Anschlusselement 74 immer in der abdichtenden Anlage am Aufnahmekörper 32. Wenn jedoch der Druck in der Druckgaspatrone 3 den normalen Maximaldruck des Gases in der Druckgaspatrone überschreitet, beispielsweise bei einem Sterilisiervorgang und der damit verbundenen Temperaturerhöhung, kann die Kraft, die von dem unter Druck stehenden Gas auf das Anschlusselement 74 ausgeübt wird und das Anschlusselement 74 in Strömungsrichtung zu verschieben sucht, so groß werden, dass entgegen der Wirkung der Tellerfeder 79 das Anschlusselement 74 von der Rückseite des Bodens des Anschlussschachtes 33 abgehoben wird, so dass nunmehr in den stromaufwärts der ersten Ringdichtung 69 gelegenen Abschnitt der Ventilkammer 65 Gas eintreten kann, das heißt es erfolgt eine Belüftung in diesem Bereich und ein Druckabbau des in der Druckgaspatrone 3 und dem anschließenden Strömungskanal herrschenden Überdruckes. Damit wirkt das Anschlusselement 74 als Sicherheitsventil, das beim Überschreiten des Maximalwertes des Druckes, der deutlich über dem normalen Betriebsdruck liegt, öffnet und diesen Überdruck abbaut.

Bei einem Sterilisiervorgang ist normalerweise der Anschluss 2 vom Anschlussstutzen 1 getrennt, so dass der Schließkörper 23 in Schließstellung steht. Dadurch herrscht stromabwärts des Ventilkörpers 68 normalerweise derselbe Druck wie stromaufwärts. Aufgrund der unterschiedlichen Querschnitte des Ventilkörpers 68 einerseits und des Anschlusselementes 74 andererseits führt dies dazu, dass das Anschlusselement 74 abdichtend am Aufnahmekörper 32 anliegt.

Bei einer Temperaturerhöhung, beispielsweise während eines Sterilisiervorgangs, steigt der Druck stromabwärts des Ventilkörpers 68 entsprechend der normalen Temperaturabhängigkeit des Druckes bei Gasen proportional zur Zunahme der Temperatur an, während auf der stromaufwärts gelegenen Seite der Druck überproportional ansteigt. Dies liegt darin, dass das Druckgas in der Druckgaspatrone in flüssiger Form vorliegt und beim Temperaturanstieg in einen überkritischen Bereich gelangt, in dem der Druck bei Temperaturerhöhung sehr viel stärker ansteigt als bei einem reinen Gas. Dieser Druckanstieg auf der stromaufwärts gelegenen Seite des Ventilkörpers und des Anschlusselementes führt schließlich zu einem Abheben des Anschlusselementes 74 von dem Aufnahmekörper 32, wenn das Verhältnis des Drucks stromaufwärts des Anschlusselementes 74 zum Druckstrom abwärts des Ventilkörpers 68 größer wird als das Flächenverhältnis, das sich durch die unterschiedlichen Querschnitte des Ventilkörpers 68 einerseits (Durchmesser D) und des Anschlusselementes 74 andererseits (Durchmesser d) ergibt.

Sowohl der Ventilkörper 68 als auch das Anschlusselement 74 werden dabei durch dieselbe Tellerfeder 79 beaufschlagt, die einmal dafür sorgt, dass normalerweise der Ventilkörper 68 in der Öffnungsstellung steht und das Anschlusselement 74 in der Dichtstellung, die Differenz zwischen der Kraft der Tellerfeder 79 einerseits und der jeweils auf den Ventilkörper 68 beziehungsweise das Anschlusselement 74 wirkenden Überdruckkraft wird dann zur Verstellung des Ventilkörpers 68 beziehungsweise des Anschlusselementes 74 verwendet.

Trotz der beschriebenen Abdichtungsmaßnahmen ist nicht ganz auszuschließen, dass im Anschlussbereich der Druckgaspatrone 3 bei der Dichtung der Dichtkappe 35 und der Dichtfläche 48 unerwünscht Gas seitlich in den Anschlussschacht 33 austreten könnte. Um einen Aufbau des Gases in diesem Bereich zu verhindern, ist es günstig, wenn die Dichtkappe 35 in ihrem seitlichen Randbereich kanalförmige Vertiefungen 80 aufweist, durch die das Gas seitlich an der Dichtkappe 35 vorbei aus dem Teil des Anschlussschachtes 33 nach außen abströmen kann, der durch das Dichtstück 45 gegenüber dem Abschnitt 59 des Anschlussschachtes 33 abgedichtet ist.

## Patentansprüche

1. Druckgasbetriebenes Instrument mit einem Anschluss für eine Druckgaspatrone (3), an dem die Druckgaspatrone (3) abgedichtet mit einem Zufuhrkanal für aus der Druckgaspatrone (3) ausströmendes Druckgas verbindbar ist, mit einem Anschlussschacht (33), in den die Druckgaspatrone (3) mit ihrem Anschlussbereich einsetzbar ist, und mit einem gegenüber der Innenwand des Anschlussschachtes (33) abgedichteten Dichtstück (45), das in dem Anschlussschacht (33) zwischen einer eingeschobenen Dichtstellung, in der es abdichtend an die Stirnfläche der in den Anschlussschacht (33) eintauchenden Druckgaspatrone (3) angedrückt wird, und einer ausgeschobenen Ruhestellung verschiebbar gelagert ist, **dadurch gekennzeichnet, dass** der Anschlussschacht (33) eine Belüftungsöffnung (61) aufweist, die mit dem stromabwärts des Dichtstücks (45) gelegenen Abschnitt (59) des Anschlussschachtes (33) in Verbindung steht, wenn das Dichtstück (45) bis zu einer zwischen Dichtstellung und Ruhestellung liegenden Belüftungsstellung aus seiner Dichtstellung entfernt ist, während das Dichtstück (45) diesen Abschnitt (59) in der Dichtstellung von der Belüftungsöffnung (61) abtrennt.

2. Instrument nach Anspruch 1, **dadurch gekennzeichnet, dass** die Druckgaspatrone (3) durch eine Haltevorrichtung (7) im Anschlussschacht (33) gehalten ist, die bei einem Herausziehen der Druckgaspatrone (3) aus dem Anschlussschacht (33) die Druckgaspatrone (3) erst völlig freigibt, wenn das Dichtstück (45) die Belüftungsstellung erreicht oder in Richtung auf die Ruhestellung überschritten hat.

3. Instrument nach Anspruch 1 oder 2, **dadurch gekennzeichnet, dass** zur Abdichtung des Dichtstücks (45) gegenüber der Innenwand des Anschlussschachtes (33) in einer Umfangsnut (56) der Innenwand eine an der Außenseite des Dichtstückes (45) anliegende Ringdichtung (55) angeordnet ist.

4. Instrument nach Anspruch 3, **dadurch gekennzeichnet, dass** das Dichtstück (45) einen Abschnitt (62) mit reduziertem Außendurchmesser aufweist, der bei Erreichen der Belüftungsstellung in den Bereich der Ringdichtung (55) gelangt und **dadurch** einen Strömungsweg (63) von dem stromabwärts des Dichtstückes (45) gelegenen Abschnitt (59) des Anschlussschachtes (33) zu der Belüftungsöffnung (61) freigibt.

5. Instrument nach Anspruch 4, **dadurch gekennzeichnet, dass** das Dichtstück (45) in dem Abschnitt (62) mit reduziertem Durchmesser mindestens eine Durchbrechung (64) aufweist, die den Zwischenraum (63) zwischen der Außenwand des Dichtstückes (45) und der Innenwand des Anschlussschachtes (33) mit dem stromabwärts des Dichtstückes (45) gelegenen Abschnitt (59) des Anschlussschachtes (33) verbindet.

6. Instrument nach Anspruch 5, **dadurch gekennzeichnet, dass** das Dichtstück (45) hülsenförmig ausgebildet ist und sein Innenraum mit dem stromabwärts des Dichtstückes (45) gelegenen Abschnitt (59) des Anschlussschachtes (33) in Verbindung steht.

7. Instrument nach Anspruch 6, **dadurch gekennzeichnet, dass** die Durchbrechung (64) als radiale Bohrung in der Wand des hülsenförmigen Dichtstückes (45) ausgebildet ist.

8. Instrument nach Anspruch 1 oder 2, **dadurch gekennzeichnet, dass** zur Abdichtung des Dichtstückes (45) gegenüber der Innenwand des Anschlussschachtes (33) in einer Umfangsnut (56a) des Dichtstückes (45) eine an der Innenwand des Anschlussschachtes (33) anliegende Ringdichtung (55a) angeordnet ist.

9. Instrument nach Anspruch 8, **dadurch gekennzeichnet, dass** in der Innenwand des Anschlussschachtes (33) eine Erweiterung (63a) angeordnet ist, die der Ringdichtung (55a) gegenüberliegt, wenn das Dichtstück (45) in der Belüftungsstellung steht, und die dabei einen Strömungsweg von dem stromabwärts des Dichtstückes (45) gelegenen Abschnitt (59) des Anschlussschachtes (33) zu der Belüftungsöffnung (61) freigibt.

10. Instrument nach Anspruch 9, **dadurch gekennzeichnet, dass** die Umfangsnut (56a) mindestens eine seitliche Ausnehmung (64a) aufweist, die die Umfangsnut (56a) auch dann mit der Belüftungsöffnung (61) verbindet, wenn in der Belüftungsstellung des Dichtstückes (45) die Ringdichtung (55a) nach außen in die Erweiterung (63a) hineingedrückt wird und damit mit Ausnahme der mindestens einen Ausnehmung (64a) der Strömungsweg von dem stromabwärts des Dichtstückes (45) gelegenen Abschnitt (59) des Anschlussschachtes (33) zu der Belüftungsöffnung (61) verschlossen wird.

11. Instrument nach einem der Ansprüche 3 bis 10, **dadurch gekennzeichnet, dass** das Dichtstück (45) stromaufwärts der Ringdichtung (55; 55a) über eine in einer Umfangsnut (58) angeordnete weitere Ringdichtung (57) abgedichtet ist.

12. Instrument nach einem der voranstehenden Ansprüche, **dadurch gekennzeichnet, dass** die Belüftungsöffnung (61) als Umfangsnut in der Innenwand des Anschlussschachtes (33) ausgebildet ist.

13. Instrument nach einem der voranstehenden Ansprüche, **dadurch gekennzeichnet, dass** der Belüftungsöffnung (61) eine Drossel (61a) zugeordnet ist.

14. Instrument nach Anspruch 13, **dadurch gekennzeichnet, dass** bei einer als Umfangsnut ausgebildeten Belüftungsöffnung (61) die Belüftung über eine aus der Umfangsnut (61) austretende, einen engen Querschnitt aufweisende Belüftungsbohrung (61a) erfolgt.

15. Instrument nach einem der voranstehenden Ansprüche, **dadurch gekennzeichnet, dass** das Dichtstück (45) von einer Feder (54) in Richtung auf die Ruhestellung belastet ist.

16. Instrument nach einem der voranstehenden Ansprüche, **dadurch gekennzeichnet, dass** das Dichtstück (45) aus einem hülsenförmigen Außenteil (47) und einem die Dichtfläche (48) für die Druckgaspatrone (3) tragenden Innenteil (46) gebildet wird und sich das Außenteil (47) abgedichtet auf dem Innenteil (46) abstützt.

17. Instrument nach einem der voranstehenden Ansprüche in Kombination mit einer Druckgaspatrone, **dadurch gekennzeichnet, dass** die Druckgaspatrone (3) in ihrem in den Anschlussschacht (33) eingeschobenen Anschlussteil (34) mindestens einen Belüftungskanal (80) aufweist, der den stromaufwärts des Dichtstückes (45) und außerhalb der Dichtstelle zwischen dem Dichtstück (45) und der Druckgaspatrone (3) gelegenen Abschnitt des Anschlussschachtes (33) zur Umgebung hin öffnet.

## Claims

1. Instrument operated using pressurised gas with a connection for a pressurised gas cartridge (3), at which the pressurised gas cartridge (3) can be connected in a sealed manner to a feed channel for pressurised gas flowing out of the pressurised gas cartridge (3), with a connection shaft (33), into which the pressurised gas cartridge (3) can be inserted by its connection region, and with a sealing piece (45), which is sealed relative to the inner wall of the connection shaft (33) and is displaceably mounted in the connection shaft (33) between an inserted sealing position, in which it is pressed in sealing manner on the end face of the pressurised gas cartridge (3) entering the connection shaft (33) and a withdrawn rest position, **characterised in that** the connection shaft (33) has a ventilation opening (61), which is connected to the portion (59) of the connection shaft (33) situated downstream of the sealing piece (45) when the sealing piece (45) is removed from its sealing position up to a ventilation position located between the sealing position and rest position, while the sealing piece (45) separates this portion (59) from the ventilation opening (61) in the sealing position.

2. Instrument according to claim 1, **characterised in that** the pressurised gas cartridge (3) is held in the connection shaft (33) by a holding device (7), which only completely frees the pressurised gas cartridge (3) when the pressurised gas cartridge (3) withdrawn from the connection shaft (33), when the sealing piece (45) has reached the ventilation position or has exceeded it in the direction of the rest position.

3. Instrument according to claim 1 or 2, **characterised in that** an annular seal (55) abutting the outside of the sealing piece (45) is arranged in a peripheral groove (56) of the inner wall to seal the sealing piece (45) relative to the inner wall of the connection shaft (33).

4. Instrument according to claim 3, **characterised in that** the sealing piece (45) has a portion (62) with a reduced external diameter, which, on reaching the ventilation position, arrives in the region of the annular seal (55) and thus frees a flow path (63) from the portion (59) of the connection shaft (33) situated downstream of the sealing piece (45) to the ventilation opening (61).

5. Instrument according to claim 4, **characterised in that** the sealing piece (45), in the portion (62) with a reduced diameter, has at least one aperture (64), which connects the intermediate space (63) between the outer wall of the sealing piece (45) and the inner wall of the connection shaft (33) to the portion (59) of the connection shaft (33) situated downstream of the sealing piece (45).

6. Instrument according to claim 5, **characterised in that** the sealing piece (45) is in the form of a sleeve and its interior is connected to the portion (59) of the connection shaft (33) situated downstream of the sealing piece (45).

7. Instrument according to claim 6, **characterised in that** the aperture (64) is configured as a radial bore in the wall of the sleeve-like sealing piece (45).

8. Instrument according to claim 1 or 2, **characterised in that** an annular seal (55a) abutting the inner wall of the connection shaft (33) is arranged in a peripheral groove (56a) of the sealing piece (45) to seal the sealing piece (45) relative to the inner wall of the connection shaft (33).

9. Instrument according to claim 8, **characterised in that** arranged in the inner wall of the connection shaft (33) is a widened portion (63a), which is located opposite the annular seal (55a) when the sealing piece (45) is in the ventilation position, and which at the same time frees a flow path from the portion (59) of the connection shaft (33) situated downstream of the sealing piece (45) to the ventilation opening (61).

10. Instrument according to claim 9, **characterised in that** the peripheral groove (56a) has at least one lateral recess (64a), which also connects the peripheral groove (56a) to the ventilation opening (61) when the annular seal (55a) is pressed outwardly into the widened portion (63a) in the ventilation position of the sealing piece (45) and therefore, with the exception of the at least one recess (64a), the flow path from the portion (59) of the connection shaft (33) situated downstream of the sealing piece (45) to the ventilation opening (61) is closed.

11. Instrument according to any one of claims 3 to 10, **characterised in that** the sealing piece (45) upstream of the annular seal (55; 55a) is sealed by a further annular seal (57) arranged in a peripheral groove (58).

12. Instrument according to any one of the preceding claims, **characterised in that** the ventilation opening (61) is configured as a peripheral groove in the inner wall of the connection shaft (33).

13. Instrument according to any one of the preceding claims, **characterised in that** a restrictor (61a) is associated with the ventilation opening (61).

14. Instrument according to claim 13, **characterised in that** with a ventilation opening (61) configured as a peripheral groove, the ventilation takes place by means of a ventilation bore (61a) emerging from the peripheral groove (61) and having a narrow cross-section.

15. Instrument according to any one of the preceding claims, **characterised in that** the sealing piece (45) is loaded by a spring (54) in the direction of the rest position.

16. Instrument according to any one of the preceding claims, **characterised in that** the sealing piece (45) is formed from a sleeve-like outer part (47) and an inner part (46) bearing the sealing face (48) for the pressurised gas cartridge (3) and the outer part (47) is supported in a sealed manner on the inner part (46).

17. Instrument according to any one of the preceding claims in combination with a pressurised gas cartridge, **characterised in that** the pressurised gas cartridge (3), in its connection part (34) inserted in the connection shaft (33), has at least one ventilation channel (80) which opens to the surroundings the portion of the connection shaft (33) situated upstream of the sealing piece (45) and outside the sealing location between the sealing piece (45) and the pressurised gas cartridge (3).

## Revendications

1. Instrument fonctionnant par gaz comprimé, comprenant un raccord pour une cartouche de gaz comprimé (3), au niveau duquel la cartouche de gaz comprimé (3) peut être reliée de manière étanche avec un canal d'alimentation destiné à du gaz comprimé s'écoulant hors de la cartouche de gaz comprimé (3), comprenant par ailleurs une coupelle de raccordement (33) dans laquelle peut être insérée la cartouche de gaz comprimé (3) avec sa zone de raccordement, et comprenant une pièce d'étanchéité (45) étanche par rapport à la paroi intérieure de la coupelle de raccordement (33) et qui est montée coulissante dans la coupelle de raccordement (33), entre une position insérée d'étanchéité, dans laquelle elle est pressée contre la surface frontale de la cartouche de gaz comprimé (3) s'engageant dans la coupelle de raccordement (33), et une position de repos d'extraction, **caractérisé en ce que** la coupelle de raccordement (33) présente une ouverture de purge (61), qui est en communication avec le tronçon (59) de la coupelle de raccordement (33) situé en aval de la pièce d'étanchéité (45), lorsque la pièce d'étanchéité (45) est éloignée de sa position d'étanchéité, jusqu'à une position de purge située entre la position d'étanchéité et la position de repos, tandis que la pièce d'étanchéité (45), dans la position d'étanchéité, isole ce tronçon (59) de l'ouverture de purge (61).

2. Instrument selon la revendication 1, **caractérisé en ce que** la cartouche de gaz comprimé (3) est maintenue dans la coupelle de raccordement (33) par un dispositif de maintien (7) qui, lors d'un retrait de la cartouche de gaz comprimé (3) de la coupelle de raccordement (33), ne libère totalement la cartouche de gaz comprimé (3) que lorsque la pièce d'étanchéité (45) a atteint la position de purge ou l'a dépassée en direction de la position de repos.

3. Instrument selon la revendication 1 ou la revendication 2, **caractérisé en ce que** pour assurer l'étanchéité de la pièce d'étanchéité (45) par rapport à la paroi intérieure de la coupelle de raccordement (33), un joint d'étanchéité annulaire (55), qui s'appuie sur le côté extérieur de la pièce d'étanchéité (45), est agencé dans une rainure périphérique (56) de ladite paroi intérieure.

4. Instrument selon la revendication 3, **caractérisé en ce que** la pièce d'étanchéité (45) présente un tronçon (62) de diamètre extérieur réduit, qui, lorsque la position de purge est atteinte, parvient dans la zone du joint d'étanchéité annulaire (55) en libérant ainsi un parcours d'écoulement (63) allant du tronçon (59) de la coupelle de raccordement (33,) situé en aval de la pièce d'étanchéité (45,) à l'ouverture de purge (61).

5. Instrument selon la revendication 4, **caractérisé en ce que** la pièce d'étanchéité (45) présente, dans le tronçon (62) de diamètre réduit, au moins un passage (64) qui fait communiquer l'espace intermédiaire (63) entre la paroi extérieure de la pièce d'étanchéité (45) et la paroi intérieure de la coupelle de raccordement (33), avec le tronçon (59) de la coupelle de raccordement (33) situé en aval de la pièce d'étanchéité (45).

6. Instrument selon la revendication 5, **caractérisé en ce que** la pièce d'étanchéité (45) est d'une configuration en forme de douille, et son espace intérieur est en communication avec le tronçon (59) de la coupelle de raccordement (33) situé en aval de la pièce d'étanchéité (45).

7. Instrument selon la revendication 6, **caractérisé en ce que** le passage (64) est réalisé sous la forme d'un alésage radial dans la paroi de la pièce d'étanchéité (45) en forme de douille.

8. Instrument selon la revendication 1 ou la revendication 2, **caractérisé en ce que** pour assurer l'étanchéité de la pièce d'étanchéité (45) par rapport à la paroi intérieure de la coupelle de raccordement (33), un joint d'étanchéité annulaire (55a), qui s'appuie sur la paroi intérieure de la coupelle de raccordement (33), est agencé dans une rainure périphérique (56a) de la pièce d'étanchéité (45).

9. Instrument selon la revendication 8, **caractérisé en ce que** dans la paroi intérieure de la coupelle de raccordement (33) est agencé un évasement (63a), qui est en regard du joint d'étanchéité annulaire (55a) lorsque la pièce d'étanchéité (45) se trouve dans la position de purge, et qui à cette occasion libère un parcours d'écoulement allant du tronçon (59) de la coupelle de raccordement (33), situé en aval de la pièce d'étanchéité (45), à l'ouverture de purge (61).

10. Instrument selon la revendication 9, **caractérisé en ce que** la rainure périphérique (56a) présente au moins un évidement latéral (64a), qui met en communication la rainure périphérique (56a) avec l'ouverture de purge (61) également lorsque dans la position de purge de la pièce d'étanchéité (45), le joint d'étanchéité annulaire (55a) est repoussé vers l'extérieur dans l'évasement (63a), en fermant ainsi, exception faite dudit au moins un évidement (64a), le parcours d'écoulement allant du tronçon (59) de la coupelle de raccordement (33), situé en aval de la pièce d'étanchéité (45), à l'ouverture de purge (61).

11. Instrument selon l'une des revendications 3 à 10, **caractérisé en ce que** la pièce d'étanchéité (45) est rendue étanche en amont du joint d'étanchéité annulaire (55 ; 55a), par l'intermédiaire d'un joint d'étanchéité annulaire supplémentaire (57) agencé dans une rainure périphérique (58).

12. Instrument selon l'une des revendications précédentes, **caractérisé en ce que** l'ouverture de purge (61) est réalisée sous forme de rainure périphérique dans la paroi intérieure de la coupelle de raccordement (33).

13. Instrument selon l'une des revendications précédentes, **caractérisé en ce qu'**à l'ouverture de purge (61) est associé un étranglement (61a).

14. Instrument selon la revendication 13, **caractérisé en ce que** dans le cas d'une ouverture de purge (61) réalisée sous forme de rainure périphérique, la purge s'effectue par l'intermédiaire d'un alésage de purge (61a) de faible section transversale et sortant de la rainure périphérique (61).

15. Instrument selon l'une des revendications précédentes, **caractérisé en ce que** la pièce d'étanchéité (45) est chargée par un ressort (54) en direction de la position de repos.

16. Instrument selon l'une des revendications précédentes, **caractérisé en ce que** la pièce d'étanchéité (45) est formée par une partie extérieure (47) en forme de douille et une partie intérieure (46) portant la surface d'étanchéité (48) pour la cartouche de gaz comprimé (3), et la partie extérieure (47) s'appuie de manière étanche sur la partie intérieure (46).

17. Instrument selon l'une des revendications précédentes, en combinaison avec une cartouche de gaz comprimé, **caractérisé en ce que** la cartouche de gaz comprimé (3) présente, dans sa partie de raccordement (34) insérée dans la coupelle de raccordement (33), au moins un canal de purge (80) qui ouvre en direction de l'environnement, le tronçon de la coupelle de raccordement (33) situé en amont de la pièce d'étanchéité (45) et à l'extérieur de la zone d'étanchéité entre la pièce d'étanchéité (45) et la cartouche de gaz comprimé (3).
